# EUROPEAN PATENT SPECIFICATION

(11) **EP 4 309 717 B1**
(45) Date of publication and mention of the grant of the patent: **29.07.2026**
(21) Application number: 22770502.7
(22) Date of filing: 15.03.2022
(51) Int. Cl.: A61M 16/16, A61M 16/10

(54) **COVER OPENING AND CLOSING COMPONENT, CONTAINER COVER, CONTAINER BODY, CONTAINER DEVICE AND VENTILATION THERAPY APPARATUS**
DECKELÖFFNUNGS- UND -SCHLIESSKOMPONENTE, BEHÄLTERDECKEL, BEHÄLTERKÖRPER, BEHÄLTERVORRICHTUNG UND BEATMUNGSTHERAPIEVORRICHTUNG
ÉLÉMENT D'OUVERTURE ET DE FERMETURE DE COUVERCLE, COUVERCLE DE RÉCIPIENT, CORPS DE RÉCIPIENT, DISPOSITIF RÉCIPIENT ET APPAREIL DE THÉRAPIE PAR VENTILATION

(30) Priority: 16.03.2021 CN 202110282018
(43) Date of publication of application: 24.01.2024
(73) Proprietor: Caremedi Technology Co., Ltd, Tianjin 301700 (CN)
(72) Inventor: LIU, Lijun, Tianjin 301700 (CN); ZHUANG, Zhi, Tianjin 301700 (CN)
(74) Representative: Grünecker Patent- und Rechtsanwälte PartG mbB
(86) International application number: PCT/CN2022/080931
(87) International publication number: WO 2022/194149

(56) References cited:
- WO-A1-2009/112552
- WO-A1-2018/103700
- CN-A- 103 562 086
- CN-A- 103 656 830
- CN-A- 113 082 443
- CN-U- 2 126 021
- CN-U- 205 434 641
- CN-U- 210 904 520
- CN-Y- 2 198 227
- US-B2- 9 211 982

## Description

### TECHNICAL FIELD

The present disclosure relates to the technical field of cover opening and closing, in particular to a cover opening and closing member, and its combination with a container cover or a container body, a container device and a ventilation therapy apparatus.

### BACKGROUND

At present, ventilation therapy equipment such as ventilators and high-flow oxygen therapy devices is vital medical equipment for patients with respiratory failure to maintain breathing, ensure ventilation, save and prolong the lives thereof, which is also an effective means that can replace human spontaneous ventilation functions currently. Therefore, the ventilation therapy equipment is widely used in respiratory failure caused by various reasons, anesthesia respiratory management during major surgery, respiratory support treatment and emergency resuscitation.

For better patient adaptability, almost all ventilators are equipped with a humidifier, allowing patients to inhale warm and humid air, reducing heat and moisture consumption in the respiratory tract. In actual use, the water stored in the humidifier has a limited volume, thus the designed volume of water can basically be used by the patient for one night. Accordingly, the humidifier has to be removed from the main body of the ventilator to add water every day. However, since it is required to perform cumbersome operations of unlocking and locking on a lock part in the humidifier every time, the customer experience is poor, and the service life of the lock part is affected.

CN 2 198 227 Y discloses a cover for a bottle with an upper cover, a neck portion on which the upper cover is hinged. The neck portion can be mounted on a bottle neck in a rotatable manner to open and close the bottle manually by rotating the cover relative to the bottle horizontally. A lever rod with a heavy weight is hinged on a fixing rod. The end of the lever rod opposite to the heavy weight is contacting the inner surface of the upper cover, so that if the bottle is pivoted to a certain direction, the upper cover can be pushed by the lever rod to open.

CN 2 126 021 U discloses another bottle cover with a lever with a rotational axis in the middle pivotably seated on a piston. The lever can be pivoted to a certain direction by a half-circular shaped weight on one end, and pulled by a spring attached to a lever on the opposite end, so that the piston can be raised or lowered to open or block a liquid passage depending on whether the bottle is tilted to a certain direction.

### SUMMARY

A first object of the present disclosure is to provide a cover opening and closing member with a simple structure. In actual use, the cover opening and closing member can be kept at a vertical position by its own weight when the container body is tilted in any direction, so that the container cover can slide upward relative to the port of the container body, and the container cover can be opened conveniently.

In view of the above object, the present disclosure provides a cover opening and closing member, including: a connection rod, a cover universal connector, a supporting universal joint and a pendulum. The cover universal connector is configured to be in universal rotation fit with a universal connection portion on a container cover, the cover universal connector includes a first universal rotation mating surface that is in universal rotation fit with a second universal rotation mating surface of the universal connection portion; the supporting universal joint is configured to be in universal rotation fit with a supporting universal cavity on a supporting frame in a container internal cavity of a container body; the cover universal connector, the supporting universal joint and the pendulum are sequentially arranged on the connection rod at intervals.

In this solution, the cover universal connector includes the first universal rotation mating surface, the supporting universal joint is in universal rotation fit with the supporting universal cavity on the supporting frame inside the container internal cavity of the container body, and the cover universal connector, the supporting universal joint and the pendulum are sequentially arranged on the connection rod at intervals. In this way, when the cover opening and closing member is actually used, the first universal rotation mating surface is in universal rotation fit with the second universal rotation mating surface, the supporting universal joint is in universal rotation fit with the supporting universal cavity, so that the cover opening and closing member is kept at a vertical position, causing the container cover to cover the cover port of the container body. On the other hand, when the container body is tilted in any direction, the connection rod is always kept at a vertical position due to the pendulum, so that the cover universal connector universally rotates relative to the universal connection portion through the universal support of the supporting frame. That is, according to the lever principle, by taking the supporting frame as a universal fulcrum, the connection rod can drive the container cover to slide upward relative to the cover port of the container body so that the container cover is opened. Therefore, as long as the container body is tilted in any direction, the container cover can be opened automatically and conveniently through the cover opening and closing member, avoiding cumbersome operations of opening and closing the cover, and improving the user experience.

Optionally, each of the cover universal connector, the supporting universal joint and the pendulum is a sphere, and the first universal rotation mating surface is a spherical surface.

Optionally, the cover universal connector and the pendulum are respectively arranged at two ends of the connection rod.

Optionally, the pendulum is provided with a heater receiving cavity for receiving a heater that is configured to enable the pendulum to heat water in the container internal cavity.

Optionally, a wire routing passage extending in an axial direction is formed inside the connection rod, and a wire passing passage is formed in the cover universal connector, and the heater receiving cavity, the wire passing passage and the wire routing passage communicate with each other.

Optionally, a heater is provided in the heater receiving cavity.

A second object of the present disclosure is to provide a combination of a cover opening and closing member and a container cover with a simple structure that in actual use, can be used in conjunction with any of the above-mentioned cover opening and closing members to be conveniently opened.

The present disclosure provides a container cover, including a cover body configured with a universal connection portion that is enabled to be in universal rotation fit with the cover universal connector of any of the cover opening and closing members described above. The universal connection portion includes a second universal rotation mating surface for universal rotation fit with the first universal rotation mating surface of the cover universal connector of any of the cover opening and closing member described above.

In this technical solution, the universal connection portion includes the second universal rotation mating surface for universal rotation fit with the first universal rotation mating surface of the cover universal connector of any of the above-mentioned cover opening and closing members described above. In this way, in actual use, the first universal rotation mating surface is in universal rotation fit with the second universal rotation mating surface, the supporting universal joint is in universal rotation fit with the supporting universal cavity, so that the cover opening and closing member is kept at a vertical position, causing the container cover to cover the cover port of the container body. On the other hand, when the container body is tilted in any direction, the connection rod is always kept at a vertical position due to the pendulum, so that the cover universal connector universally rotates relative to the universal connection portion through universal support of the supporting frame. That is, according to the lever principle, by taking the supporting frame as a universal fulcrum, the connection rod can drive the container cover to slide upward relative to the cover port of the container body so that the container cover is opened. Therefore, as long as the container body is tilted in any direction, the container cover can be opened automatically and conveniently through the cover opening and closing member, avoiding cumbersome operations of opening and closing the cover, and improving the user experience.

Optionally, the universal connection portion is a spherical cavity with an inlet/outlet, and the second universal rotation mating surface is an inner spherical surface of the spherical cavity.

Optionally, the universal connection portion includes a wire passing passage.

Optionally, circumferential edges of the cover body include a ring-shaped convex spherical segment, and the ring-shaped convex spherical segment is configured to be in universal sliding fit with a ring-shaped concave spherical segment on edges of a cover port of a container body.

Optionally, the circumferential edges of the cover lid include an annular sealing ring, and the annular sealing ring includes the ring-shaped convex spherical segment.

Further, the cover body includes an air exhaust passage and an air intake passage for feeding air into the container internal cavity of the container body.

A third object of the present disclosure is to provide a combination of a cover opening and closing member and a container body with a simple structure that in actual use, can be used in conjunction with any of the above-mentioned cover opening and closing members to conveniently open the container cover.

The present disclosure provides a container body. The container body includes a container internal cavity with a cover port, a supporting frame is arranged in the container internal cavity, the supporting frame includes a supporting universal cavity that is enabled to be in universal rotation fit with the supporting universal joint of any of the cover opening and closing members described above, the supporting universal cavity includes openings at both ends in the axial direction.

In this technical solution, the supporting frame in the container internal cavity includes the supporting universal cavity for universal rotation fit with the supporting universal joint of any of the above-mentioned cover opening and closing members, and the supporting universal cavity includes openings at both axial ends so that the connection rod of any of the above-mentioned cover opening and closing members passes through the openings at both ends. In this way, in actual use, the first universal rotation mating surface is in universal rotation fit with the second universal rotation mating surface of the container cover, the supporting universal joint is in universal rotation fit with the supporting universal cavity, so that the cover opening and closing member is kept at a vertical position, causing the container cover to cover the cover port of the container body. On the other hand, when the container body is tilted in any direction, the connection rod is always kept at a vertical position due to the pendulum, so that the cover universal connector universally rotates relative to the universal connection portion through the universal support of the supporting frame. That is, according to the lever principle, by taking the supporting frame as a universal fulcrum, the connection rod can drive the container cover to slide upward relative to the cover port of the container body so that the container cover is opened. Therefore, as long as the container body is tilted in any direction, the container cover can be opened automatically and conveniently through the cover opening and closing member, avoiding cumbersome operations of opening and closing the cover, and improving the user experience.

Optionally, the supporting universal cavity is a spherical frustum cavity.

Optionally, the supporting frame includes a plurality of radial supporting rods, that are spaced from each other in a circumferential direction, wherein radial inner ends of the plurality of radial supporting rods are connected together to form a center connection portion, and radial outer ends of the plurality of radial supporting rods are connected to an inner surface of the container internal cavity, the supporting universal cavity is formed on the center connection portion.

Optionally, edges of the openings at two ends of the supporting universal cavity are respectively formed with a cutout recess.

Optionally, edges of the cover port include the ring-shaped concave spherical segment that is configured to be in universal sliding fit with the ring-shaped convex spherical segment at the circumferential edges of the container cover.

Optionally, a bottom wall and a ring-shaped side wall of the container body are connected by a ring-shaped convex cambered wall, and the convex cambered wall includes a convex cambered outer surface that is configured to be in contact with a concave cambered inner surface of a built-in heating cavity on a heating base of the container, and shapes of the convex cambered outer surface and the concave cambered inner surface are matched with each other.

Further, a fourth object of the present disclosure is to provide a container device with a simple structure that can easily open the container cover in practical use.

In view of this, the present disclosure provides a container device, including any of the cover opening and closing members described above, any of the container covers described above, and any of the container bodies described above, wherein the first universal rotation mating surface is in universal rotation fit with the second universal rotation mating surface; the supporting universal joint is in universal rotational fit with the supporting universal cavity; the cover opening and closing member is enabled to be at a vertical position to cover the container cover on the cover port of the container body; when the container body is tilted in any direction, the connection rod is always kept at the vertical position by the pendulum, so that the cover universal connector rotates relative to the universal connection portion through the universal support of the supporting frame, driving the container cover to slide upward relative to the cover port of the container body to be opened.

Thus, as stated above, the first universal rotation mating surface is in universal rotation fit with the second universal rotation mating surface, the supporting universal joint is in universal rotation fit with the supporting universal cavity, so that the cover opening and closing member is kept at a vertical position, causing the container cover to cover the cover port of the container body. On the other hand, when the container body is tilted in any direction, the connection rod is always kept at a vertical position due to the pendulum, so that the cover universal connector universally rotates relative to the universal connection portion through the universal support of the supporting frame. That is, according to the lever principle, by taking the supporting frame as a universal fulcrum, the connection rod can drive the container cover to slide upward relative to the cover port of the container body so that the container cover is opened. Therefore, as long as the container body is tilted in any direction, the container cover can be opened automatically and conveniently through the cover opening and closing member, avoiding cumbersome operations of opening and closing the cover, and improving the user experience.

Optionally, the container device includes a heating base, and the heating base includes a built-in heating cavity with an upward opening, the container body is enabled to be embedded downward in the built-in heating cavity.

Optionally, an inner circumference surface of the built-in heating cavity is provided with a plurality of elastic stoppers that are spaced in the circumferential direction, so that the container body, when embedded in the built-in heating cavity, squeezes the plurality of elastic stoppers.

Optionally, a horizontal inner bottom surface and a vertical ring-shaped inner side surface of the built-in heating cavity are connected by a ring-shaped concave cambered inner surface that is concave inward, and the concave cambered inner surface is in contact with the convex cambered outer surface of the convex cambered wall of the container body with the shapes thereof being matched.

Optionally, the container device is a humidifier.

Optionally, a power device is arranged inside the heating base, the power device communicates with the container internal cavity through a gas passage to feeding air into the container internal cavity so that the container device functions as a ventilator.

Finally, the present disclosure provides a ventilation therapy apparatus including any container device described above.

Other features and advantages of the present disclosure will be described in detail in the detailed description below.

### BRIEF DESCRIPTION OF THE DRAWINGS

The accompanying drawings which constitute a part of the description are used to provide a further understanding of the present disclosure, and are used to explain the present application in conjunction with specific embodiments described below, without limiting the present application. In the drawings:
FIG. 1 is a schematic perspective view of a container device viewed from a viewing angle provided by a specific embodiment of the present disclosure;
FIG. 2 is a schematic perspective view of the container device shown in FIG. 1 viewed from another viewing angle;
FIG. 3 is an exploded view of the container device shown in FIG. 1;
FIG. 4 is an exploded view of the container device shown in FIG. 1 in a sectional view;
FIG. 5 is a schematic cross-sectional structural view of the container device shown in FIG. 1;
FIG. 6 is a schematic cross-sectional structural view of another container device provided by a specific embodiment of the present disclosure;
FIG. 7 is a schematic cross-sectional view of the container device shown in FIG. 6 with a heating base being removed;
FIG. 8 is a schematic structural diagram illustrating that a container cover is opened when the container device shown in FIG. 7 is tilted;
FIG. 9 is a schematic perspective view of a cover opening and closing member in the container device shown in FIG. 3;
FIG. 10 is a schematic cross-sectional view of FIG. 9;
FIG. 11 is a schematic cross-sectional view of the container cover in the container device shown in FIG. 3;
FIG. 12 is a schematic structural diagram illustrating a container body of the container device shown in FIG. 3;
FIG. 13 is a schematic cross-sectional view of FIG. 12;
FIG. 14 is a schematic cross-sectional view of the heating base in the container device shown in FIG. 3; and
FIG. 15 is a schematic diagram illustrating the container body, when embedded in a built-in heating cavity in the container device shown in FIG. 3, squeezes an elastic stopper.

Reference sign list
1-connection rod, 2-cover universal connector, 3-supporting universal joint, 4-pendulum, 5-universal connection portion, 6-first universal rotation mating surface, 7-second universal rotation mating surface, 8-supporting frame, 9-supporting universal cavity, 10-wire routing passage, 11-wire passing passage, 12-cover body, 13 -inlet/outlet, 14-spherical cavity, 15-ring-shaped convex spherical segment, 16-container body, 17-ring-shaped concave spherical segment, 18-annular sealing ring, 19-air exhaust passage, 20-air intake passage, 21-container internal cavity, 22-radial supporting rod, 23-center connection portion, 24-cutout recess, 25-bottom wall, 26-side wall, 27-convex cambered wall, 28-convex cambered outer surface, 29-heating base, 30-built-in heating cavity, 31-concave cambered inner surface, 32-cover opening and closing member, 33-container cover, 34-elastic stopper, 35-horizontal inner bottom surface, 36-vertical inner side surface, 37-power device, 38- gas passage, 39-anti-skid supporting pad

### DETAILED DESCRIPTION

The specific implementation of the present disclosure will be described in detail below in conjunction with the accompanying drawings. It should be understood that the specific implementations described here are only used to illustrate and explain the present application, and are not intended to limit the present application.

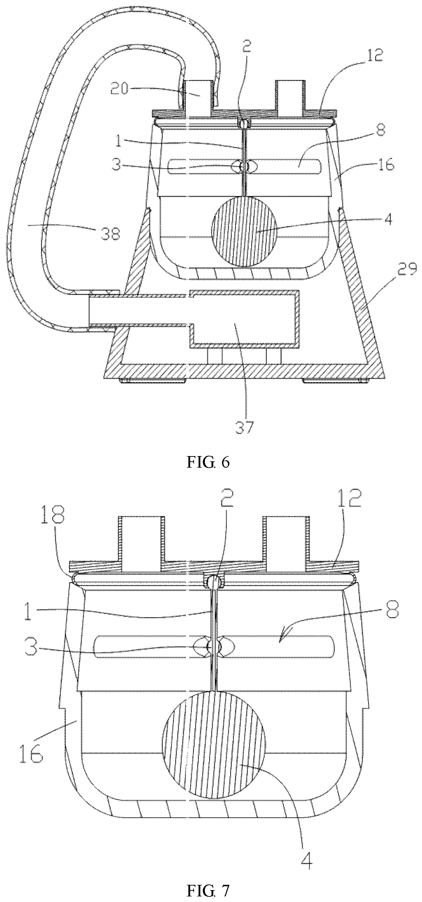

Referring to FIG. 7, FIG. 8, and FIG. 9 to FIG. 13, a first object of the present disclosure is to provide a cover opening and closing member 32, including a connection rod 1, a cover universal connector 2, a supporting universal joint 3 and a pendulum 4. The cover universal connector 2 is used for universal rotation fit with a universal connection portion 5 on the container cover, and the cover universal connector 2 includes a first universal rotation mating surface 6 for universal rotation fit with a second universal rotation mating surface 7 of the universal connection portion 5. The supporting universal joint 3 is used for universal rotation fit with a supporting universal cavity 9 on the supporting frame 8 of a container internal cavity of the container body. The cover universal connector 2, the supporting universal joint 3 and the pendulum 4 are sequentially arranged on the connection rod 1 at intervals.

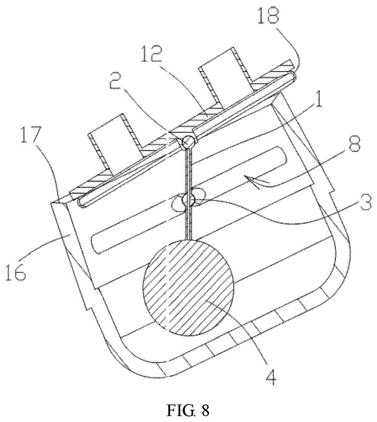

In this technical solution, the cover universal connector 2 includes the first universal rotation mating surface 6, the supporting universal joint 3 is used for universal rotation fit with the supporting universal cavity 9 on the supporting frame 8 of the container internal cavity 21 of the container body 16, and the cover universal connector 2, the supporting universal joint 3 and the pendulum 4 are arranged on the connection rod 1 at intervals. In this way, when the cover opening and closing member 32 is actually used, by the universal rotation fit between the first universal rotation mating surface 6 and the second universal rotation mating surface 7 on the container cover as well as the universal rotation fit between the supporting universal joint 3 and the supporting universal cavity 9, the cover opening and closing member 32 is kept at a vertical position, so that the container cover 33 covers the cover port of the container body 16, as shown in FIG. 7. On the other hand, when the container body 16 is tilted in any direction, with reference to FIG. 8, the connection rod 1 is always kept at a vertical position due to the pendulum 4, so that the connection rod 1 is universally supported by the supporting frame 8 and the cover universal connector 2 universally rotates relative to the universal connection portion 5. That is, according to the lever principle, by taking the supporting frame 8 as a universal fulcrum, the connection rod 1 can drive the container cover 33 to slide upward relative to the port of the container body 16 so that the container cover 33 is opened. Therefore, as long as the container body 16 is tilted in any direction, the container cover 33 can be opened automatically and conveniently through the cover opening and closing member 32, avoiding cumbersome operations of opening and closing the cover, and improving the user experience.

In addition, in the cover opening and closing member, the cover universal connector 2 may have various structural forms as long as the cover universal connector 2 can realize the universal rotation fit with the universal connection portion 5 on the container cover. For example, one structural form of the cover universal connector 2 is a spherical frustum, which is a geometric body surrounded by a spherical zone and two parallel planes intercepting the spherical zone, that is, a part of a sphere sandwiched between two parallel planes intercepting the sphere. In this way, the first universal rotation mating surface 6 is an outer spherical surface of the spherical zone of the spherical frustum, that is, a ring-shaped spherical surface sandwiched between two parallel planes. When the cover universal connector 2 is a spherical frustum, the universal connection portion 5 may be a spherical frustum shaped cavity, that is, an inner contour of the spherical frustum shaped cavity is an outer contour of the spherical frustum. At this time, the second universal rotation mating surface 7 is a ring-shaped part of the concave spherical surface of the spherical frustum shaped cavity. In this way, the spherical frustum can squeeze the opening of the spherical frustum shaped cavity and enter the spherical frustum shaped cavity, so that the ring-shaped part of the concave spherical surface and the ring-shaped spherical surface of the spherical frustum form universal rotation fit.

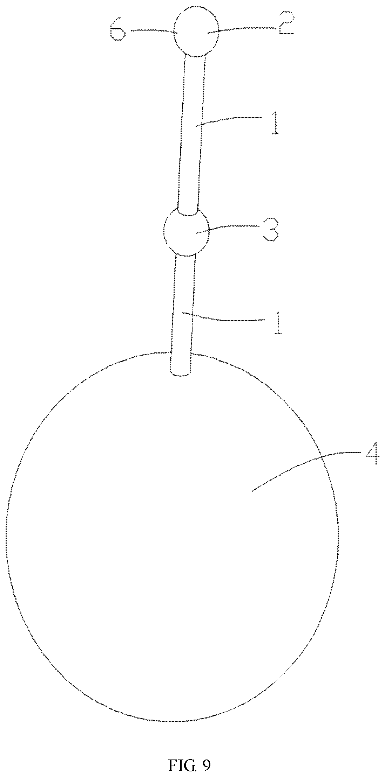

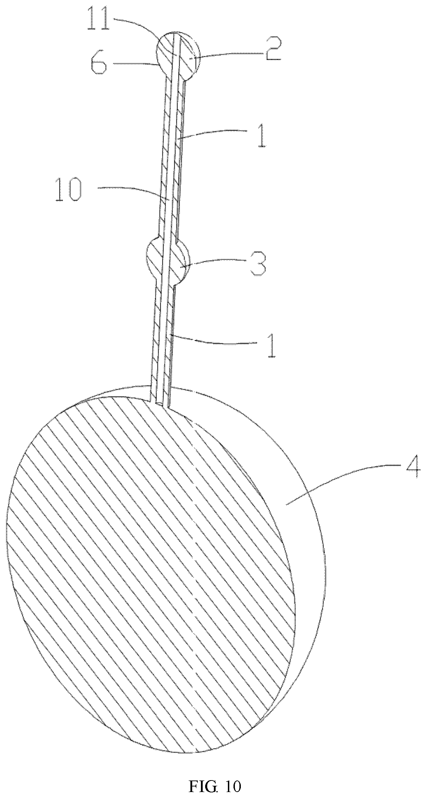

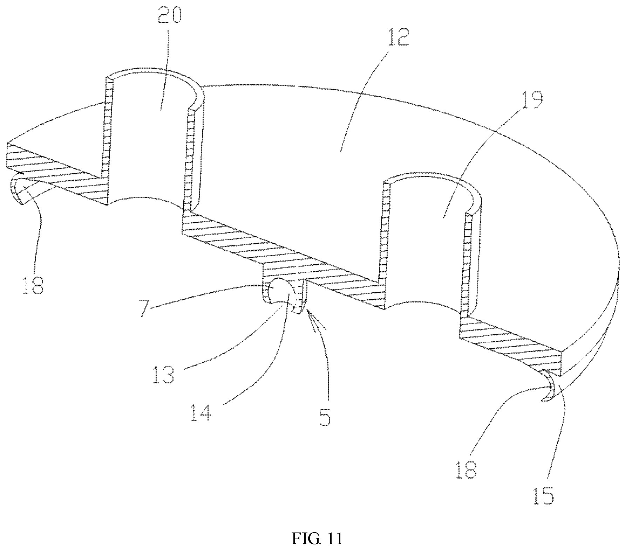

Alternatively, referring to FIG. 9 and FIG. 10, in another structural form of the cover universal connector 2, the cover universal connector 2 is a sphere, thus the first universal rotation mating surface 6 is a spherical surface. Accordingly, referring to FIG. 11, the universal connection portion 5 is a spherical cavity 14 with an inlet/outlet 13, and the second universal rotation mating surface 7 is the inner spherical surface of the spherical cavity 14. Accordingly, the sphere may squeeze the inlet/outlet 13 and enter the spherical cavity 14, so that the spherical surface and the inner spherical surface form universal rotation fit.

In an optional embodiment, the cover universal connector 2 may include a spherical frustum shaped cavity or a spherical cavity, and correspondingly, the universal connection portion 5 may be a spherical frustum or a sphere. Accordingly, the spherical frustum or the sphere may squeeze the opening of the spherical frustum shaped cavity or the spherical cavity and enter the spherical frustum shaped cavity or spherical cavity to form universal rotation fit.

Of course, alternatively, the cover universal connector 2 and the universal connection portion 5 may include at least a part of spherical surfaces that implement the universal rotation fit, or the cover universal connector 2 and the universal connection portion 5 may include cambered surfaces that implement the universal rotation fit.

In addition, the pendulum 4 may have any structure and shape. For example, the pendulum 4 may be a block or a sphere.

In addition, the supporting universal joint 3 may have various structural forms, as long as it can form universal rotation fit with the supporting universal cavity 9 on the supporting frame 8 inside the internal cavity of the container body. For example, the supporting universal joint 3 may be a cylinder. At this time, the supporting universal cavity 9 is a cylindrical cavity, as long as the inner diameter of the cylindrical cavity is greater than the outer diameter of the cylinder, and the supporting universal cavity may form a lever support of the connection rod when the connection rod is universally tilted. Alternatively, the supporting universal joint 3 may be a spherical frustum or a sphere, and the supporting universal cavity 9 may be a spherical frustum shaped cavity. Two ends of the spherical frustum shaped cavity are opened so that the connection rod 1 can pass through. In this way, the spherical frustum shaped cavity forms a stable and reliable lever universal support for the connection rod when the connection rod is tilted. As another example, the supporting universal joint 3 may include a ring-shaped convex cambered surface, and the supporting universal cavity 9 may be a concave cambered cavity, and both ends of the concave cambered cavity are opened to facilitate the passage of the connection rod 1.

In addition, in the cover opening and closing member, the cover universal connector 2 and the pendulum 4 may be provided at positions of the connection rod 1 away from ends of the connection rod at a set distance, that is, the cover universal connector 2 and the pendulum 4 keep a distance relative to the two ends of connection rod 1 respectively. Alternatively, in another embodiment, referring to FIG. 9 and FIG. 10, the cover universal connector 2 and the pendulum 4 are respectively disposed at both ends of the connection rod 1 so that the cover universal connector 2 can be better fitted in the shape of the universal rotation. Moreover, it is also convenient for the cover universal connector 2 and the pendulum 4 to be connected to the ends of the connection rod 1 respectively. For example, the cover universal connector 2 and the pendulum 4 are respectively formed with insertion holes, so that the two ends of the connection rod 1 can be inserted and fixed into the insertion holes on the cover universal connector 2 and the pendulum 4 respectively.

In addition, in an embodiment, the pendulum 4 may be located in the internal cavity of the container, and the pendulum 4 may be fully utilized to heat the water in the internal cavity of the container. To this end, the pendulum 4 is provided with a heater receiving cavity for receiving a heater that is configured to cause the pendulum to heat the water in the internal cavity of the container. Thus, in actual use, the heater may be arranged in the heater receiving cavity. After being energized, the heater may cause the pendulum 4 to heat the water in the internal cavity of the container. Of course, the pendulum 4 may be provided with a sealing lid. The sealing lid may seal and cover the heater containing cavity after the heater is placed in the heater receiving cavity.

Of course, in an embodiment, wires connected to the heater may pass through the pendulum 4. Alternatively, in another embodiment, referring to FIG. 10, a wire routing passage 10 extending in an axial direction is formed inside the connection rod 1, and a wire passing passage 11 is formed in the cover universal connector 2, and the heater receiving cavity, the wire passing passage 11 and the wire routing passage 10 communicate with each other. Then, in actual use, wires may be disposed in and pass through the wire routing passage 10 and the wire passing passage 11, and enter the heater receiving cavity to be connected to the heater disposed in the heater receiving cavity.

In an optional embodiment, a heater is disposed in the heater receiving cavity. Alternatively, in an optional embodiment, the heater may be directly packaged in the pendulum 4, for example by integral injection molding.

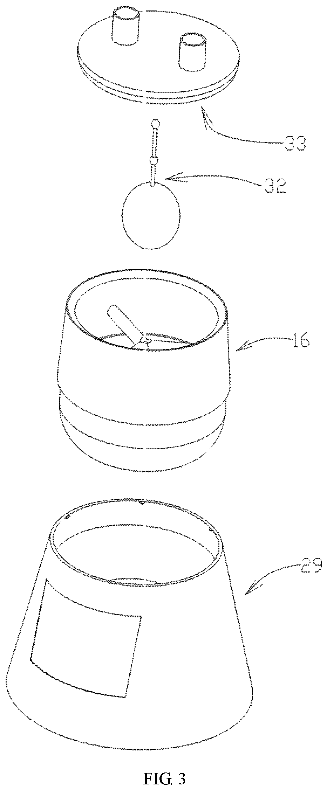

Further, a second object of the present disclosure is to provide a container cover 33. Referring to FIG. 3, FIG. 4 and FIG. 11, the container cover 33 includes a cover body 12, on which is provided with the universal connection portion 5 configured to form universal rotation fit with the cover universal connector 2 of any of the cover opening and closing members described above. The universal connection portion 5 includes the second universal rotation mating surface for the universal rotation fit with the first universal rotation mating surface 6 of the cover universal connector 2 of any of the cover opening and closing members described above.

In this technical solution, the universal connection portion 5 includes the second universal rotation mating surface 7 for universal rotation fitting with the first universal rotation mating surface 6 of the cover universal connector 2 of any of the above-mentioned cover opening and closing members 32. Accordingly, in actual use, the first universal rotation mating surface 6 and the second universal rotation mating surface 7 on the container cover are in universal rotation fit, the supporting universal joint 3 and the supporting universal cavity 9 are in universal rotation fit, so that the cover opening and closing member 32 is kept at a vertical position, causing the container cover 33 to cover the port of the container body 16; and, when the container body 16 is tilted in any direction, the connection rod 1 is always kept at a vertical position due to the pendulum 4, so that the connection rod 1 is universally supported by the supporting frame 8 and the cover universal connector 2 universally rotates relative to the universal connection portion 5, that is, according to the lever principle, by taking the supporting frame 8 as an universal fulcrum, the connection rod 1 can drive the container cover 33 to slide upward relative to the port of the container body 16 so that the container cover 33 is opened. Therefore, as long as the container body 16 is tilted in any direction, the container cover 33 can be opened automatically and conveniently through the cover opening and closing member 32, avoiding cumbersome operations of opening and closing the cover, and improving the user experience.

In addition, as mentioned above, in an embodiment, the universal connection portion 5 may be a spherical frustum or a sphere, or in another embodiment, referring to FIG. 11, the universal connection portion 5 is a spherical cavity 14 with an inlet/outlet 13, and the second universal rotation mating surface 7 is the inner spherical surface of the spherical cavity 14. Thus, the sphere/spherical frustum can be squeezed into the inlet/outlet 13 and enter into the spherical cavity 14, so that a universal rotational fit is formed between the sphere/spherical frustum and the spherical cavity 14.

In addition, the universal connection portion 5 includes a wire passing passage, so that in actual use, wires may pass through the wire passing passage to enter the wire routing passage 10 and the wire passing passage 11, and enter the heater receiving cavity and be connected with the heater in the heating body receiving cavity.

Furthermore, in an embodiment, when the container body 16 is tilted in any direction, in order to further facilitate the automatic and convenient opening of the container cover 33 under the leverage of the connection rod 1, with reference to FIG. 11, circumferential edges of the cover body 12 include a ring-shaped convex spherical segment 15. The ring-shaped convex spherical segment 15 is used for universal sliding fit with a ring-shaped concave spherical segment 17 on edges of the port of the container body 16. In this way, when the container body 16 is tilted in any direction, under the leverage of the connection rod 1 with the supporting frame 8 as the fulcrum, the ring-shaped convex spherical segment 15 of the cover body 12 is in universal sliding fit with the ring-shaped concave spherical segment 17, so that the container cover is easier to slide upward relative to the port of the container body 16. At this time, the remaining water in the container internal cavity may be poured out from the gap between the cover body 12 and the container body.

Furthermore, in order to improve the sealing performance of the container cover and the cover port of the container body, referring to FIG. 11, the circumferential edges of the cover body 12 include an annular sealing ring 18, and the annular sealing ring 18 includes the ring-shaped convex spherical segment 15. The annular sealing ring 18 may be a separate component connected with the cover body 12, or the annular sealing ring 18 may be integrally formed with the cover body 12 by encapsulation molding, and when the container cover slides upward relative to the opening of the container body 16 to be opened, the annular sealing ring 18 loses its sealing function on the cover port of the container body, so that the remaining water in the container internal cavity may be poured from the gap between the annular sealing ring 18 and the cover port.

Furthermore, referring to FIG.11, in an embodiment, the cover 12 includes an air exhaust passage 19 and an air intake passage 20 for feeding air into the container internal cavity of the container body, so that when the container cover covers the port of the container body, the steam generated when the water in the container cavity is heated will escape from the air exhaust passage 19. Furthermore, the air intake passage 20 may be connected with a power device 37 through a gas passage 38. Thus, when running, the power device 37 may increase the pressure in the container internal cavity, so that the steam in the container internal cavity may be quickly discharged, thereby realizing the function of the ventilator by the container device.

A third object of the present disclosure is to provide a container body. Referring to FIG. 3 to FIG. 8, FIG. 12 and FIG. 13, the container body 16 includes a container internal cavity 21 with a cover port, and the supporting frame 8 is provided in the container internal cavity 21. The supporting frame 8 includes a supporting universal cavity 9 used for universal rotation fit with the supporting universal joint 3 of any of the above-mentioned cover opening and closing members. Th supporting universal cavity 9 includes openings at both axial ends.

Accordingly, in this technical solution, the supporting frame 8 in the internal cavity 21 includes the supporting universal cavity 9 for universal rotation fit with the supporting universal joint 3 of any of the above-mentioned cover opening and closing member 32, and the supporting universal cavity 9 includes openings at both axial ends, so that the connection rod 1 of any of the above-mentioned cover opening and closing members 32 passes through the openings at both ends. Accordingly, in actual use, the first universal rotation mating surface 6 and the second universal rotation mating surface 7 on the container cover are in universal rotation fit, the supporting universal joint 3 and the supporting universal cavity 9 are in universal rotation fit, so that the cover opening and closing member 32 is kept at a vertical position, causing the container cover 33 to cover the cover port of the container body 16; and, when the container body 16 is tilted in any direction, the connection rod 1 is always kept at a vertical position due to the pendulum 4, so that the connection rod 1 is universally supported by the supporting frame 8 and the cover universal connector 2 universally rotates relative to the universal connection portion 5, that is, according to the lever principle, by taking the supporting frame 8 as an universal fulcrum, the connection rod 1 can drive the container cover 33 to slide upward relative to the cover port of the container body 16 so that the container cover 33 is opened. Therefore, as long as the container body 16 is tilted in any direction, the container cover 33 can be opened automatically and conveniently through the cover opening and closing member 32, avoiding cumbersome operations of opening and closing the cover, and improving the user experience.

Furthermore, as mentioned above, in an embodiment, the supporting universal joint 3 may be a cylinder. In this case, the supporting universal cavity 9 is a cylindrical cavity, as long as the inner diameter of the cylindrical cavity is larger than the outer diameter of the cylinder, and a lever support is formed for the connection rod when the connection rod is tilted universally. Alternative, in an optional embodiment, the supporting universal joint 3 may be a spherical frustum or a sphere, and the supporting universal cavity 9 may be a spherical frustum cavity. The two ends of the spherical frustum cavity are opened so that the connection rod 1 can pass through. In this way, a stable and reliable lever universal support for the connection rod can be better formed when the connection rod is tilted in any direction. As another example, the supporting universal joint 3 may include a ring-shaped convex cambered surface, and the supporting universal cavity 9 may be a concave cambered cavity, and both ends of the concave cambered cavity are opened to facilitate the passage of the connection rod 1.

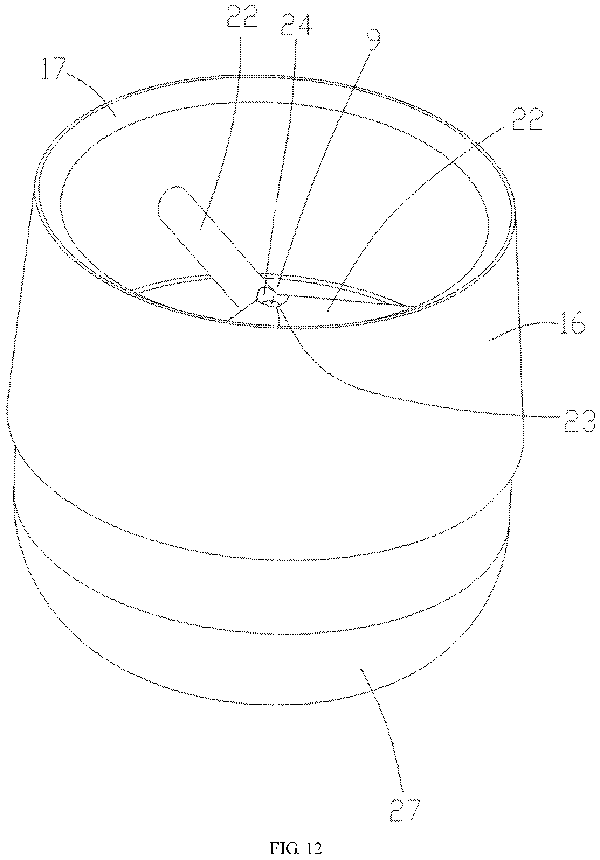

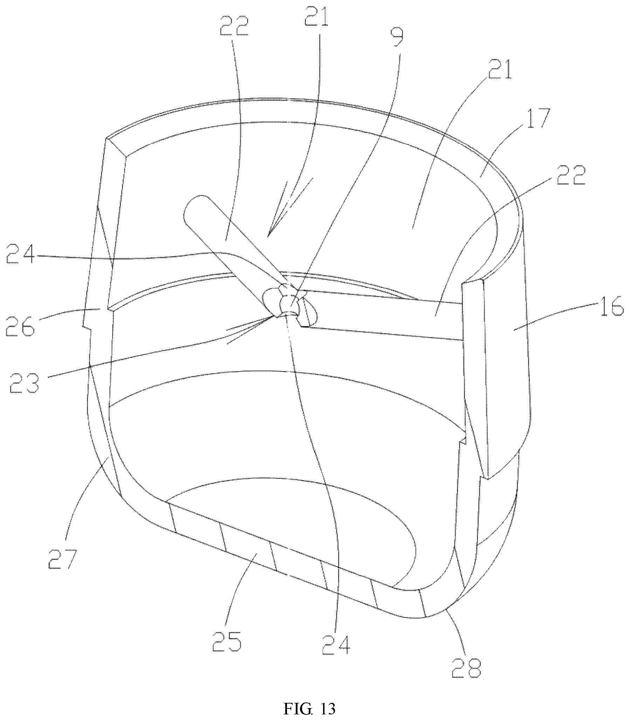

Furthermore, in the container body 16, the supporting frame 8 may have various structural forms. However, it should be noted that the supporting frame 8 may adopt any structural form, as long as it can be provided in the container internal cavity and form the supporting universal cavity 9. For example, in a structural form, the supporting frame 8 may be a diameter rod, and two ends of the diameter rod are respectively connected to the inner circumference surface of the container internal cavity, and the supporting universal cavity 9 may be formed on the diameter rod. Alternatively, in another structural form, referring to FIG. 12 and FIG.13, the supporting frame 8 includes a plurality of radial supporting rods 22 that are spaced from each other in a circumferential direction. The radial inner ends of the plurality of radial supporting rods 22 are connected together to form a center connection portion 23. The radial outer ends of the plurality of radial supporting rods 22 are connected to the inner surface of the container internal cavity 21. The supporting universal cavity 9 is formed on the center connection portion 23. In this way, through a plurality of radial supporting rods 22 that are spaced in the circumferential direction and connected together at the radial inner ends thereof, the stability and reliability of the supporting frame 8 can be improved, so that the connection rod 1 can have a more stable and reliable lever fulcrum. Accordingly, the container cover may slide upward more smoothly to be opened.

Furthermore, in order to further expand the universal rotation range of the connection rod 1, referring to FIG. 12 and FIG. 13, the edges of the openings at two ends of the supporting universal cavity 9 are respectively formed with a cutout recess 24. In this way, by the cutout recesses 24, the restriction on the universal rotation of the connection rod 1 can be accordingly reduced, so that the connection rod 1 has a further increased gimbal rotation tilt angle relative to the side surface. The cutout recess 24 may have various shapes, for example, it may be an inner concave portion or a tilted plane.

Furthermore, when the container body 16 is tilted in any direction, in order to further facilitate the container cover 33 to be automatically and conveniently opened under the leverage of the connection rod 1, referring to FIG.12 and FIG. 13, the edge of the cover port includes the ring-shaped concave spherical segment 17 for universal sliding fit with the ring-shaped convex spherical segment 15 at the circumferential edges of the container cover. Thus, when the container body 16 is tilted in any direction, under the leverage of the connection rod 1 with the supporting frame 8 as the fulcrum, the ring-shaped convex spherical segment 15 of the cover body 12 will slide relative to the ring-shaped concave spherical segment 17 in any direction, so that the container cover easily slide upward relative to the cover port of the container body 16 to be opened. At this time, the remaining water in the container internal cavity can be poured out from the gap between the cover body 12 and the container body.

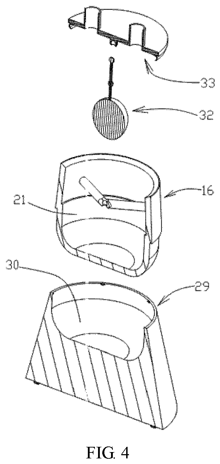

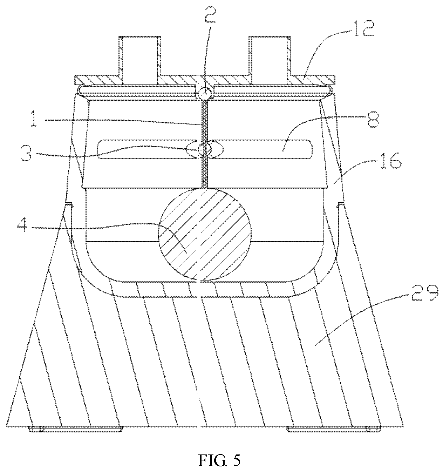

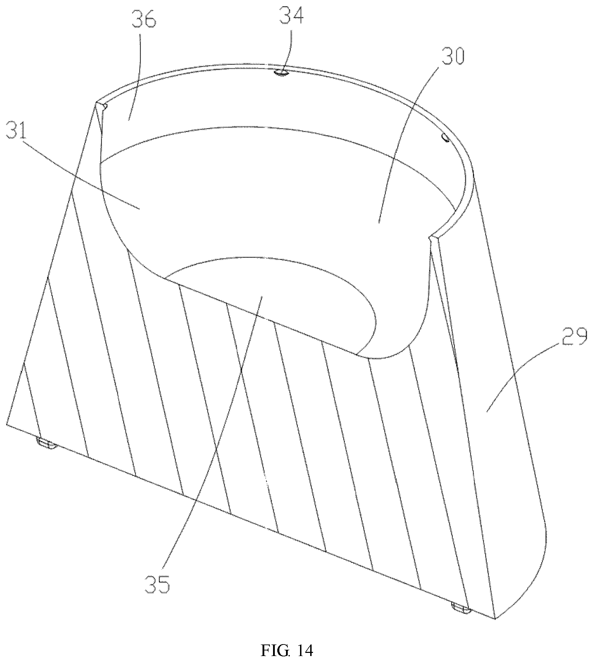

Furthermore, the container body 16 may have various shapes. For example, in one shape, referring to FIG. 12 and FIG. 13, a bottom wall 25 and a ring-shaped side wall 26 of the container body 16 are connected by a ring-shaped convex cambered wall 27, and the convex cambered wall 27 includes a convex cambered outer surface 28 that is configured to contact with a concave cambered inner surface 31 (referring to FIG. 14) of a built-in heating cavity 30 on a heating base 29 of the container in a manner of shape fitting. In this way, in actual use, referring to FIG. 4 and FIG. 5, the container body 16 may be embedded in the built-in heating cavity 30 on the heating base 29, so that the convex cambered outer surface 28 is in contact with the concave cambered inner surface 31 of the built-in heating cavity 30 on the heating base 29 of the container and the shapes thereof are matched. Accordingly, the convex cambered outer surface 28 and the concave cambered inner surface 31 that are contacted and matched in shapes can make the heating of the container body 16 more uniform and improve the heating efficiency.

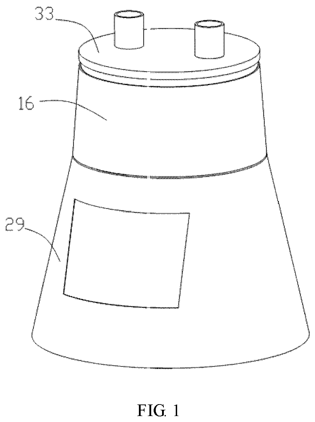

Furthermore, a fourth object of the present disclosure is to provide a container device. Referring to FIG. 1 to FIG. 8, the container device includes any cover opening and closing member 32 described above, any container cover 33 described above, and any container body 16 described above. The first universal rotation mating surface 6 and the second universal rotation mating surface 7 are in a universal rotation fit, and the supporting universal joint 3 and the supporting universal cavity 9 are in a universal rotation fit, so that the cover opening and closing member is in a vertical position to cover the container cover 33 on the cover port of the container body 16. When the container body 16 is tilted in any direction, the pendulum 4 always maintains the connection rod 1 at the vertical position, so that the cover universal connector 2 rotates relative to the universal connector 5 through the universal support of the supporting frame 8, driving the container cover 33 to slide upward relative to the cover port of the container body 16 to be opened.

Thus, as mentioned above, the first universal rotation mating surface 6 and the second universal rotation mating surface 7 on the container cover are in universal rotation fit, the supporting universal joint 3 and the supporting universal cavity 9 are in universal rotation fit, so that the cover opening and closing member 32 is kept at a vertical position, causing the container cover 33 to cover the port of the container body 16, as shown in FIG.7. On the other hand, when the container body 16 is tilted in any direction, with reference to FIG. 8, the connection rod 1 is always kept in the vertical position due to the pendulum 4, so that the connection rod 1 is universally supported by the supporting frame 8 to drive the cover universal connector 2 to universally rotate relative to the universal connecting portion 5. That is, according to the lever principle, by taking the supporting frame 8 as a universal fulcrum, the connection rod 1 can drive the container cover 33 to slide upward relative to the cover port of the container body 16 so that the container cover 33 is opened. Therefore, as long as the container body 16 is tilted in any direction, the container cover 33 can be opened automatically and conveniently through the cover opening and closing member 32, so that water in the container cavity can be poured out completely, avoiding cumbersome operations of opening and closing the cover, and improving the user experience.

Moreover, the container device is also portable, improving the convenience of carrying.

Furthermore, referring to FIG. 3, FIG. 4 and FIG. 14, the container device may include the heating base 29, and the heating base 29 includes a built-in heating cavity 30 with an upward opening, the container body 16 can be embedded downward in the built-in heating cavity 30. In this way, the container body 16 is provided above the heating base 29 of the container device in positions, and a lower half of the container body 16 can be embedded in the built-in heating cavity 30. Thus, due to the weight of the container cover and the container body, the container cover and the container body naturally come into contact with the built-in heating cavity 30 without hooks or locks, so that the container body 16 can be easily accessed to add water and pour water. However, in actual use, the water in the container internal cavity, the container cover, and the container body falls into the built-in heating cavity 30 by its own weight. In an embodiment, the cavity side wall and the cavity bottom wall of the built-in heating cavity 30 may both have the heating function, and the bottom wall 25 and ring-shaped side wall 26 of the lower half of the container body both have a heat reception function. Further, the pendulum 4 may also have the heating function, that is, the cavity side wall and cavity bottom wall of the built-in heating cavity 30 and the pendulum 4 may emit heat at the same time, thereby obtaining better use comfort and faster instantaneous availability.

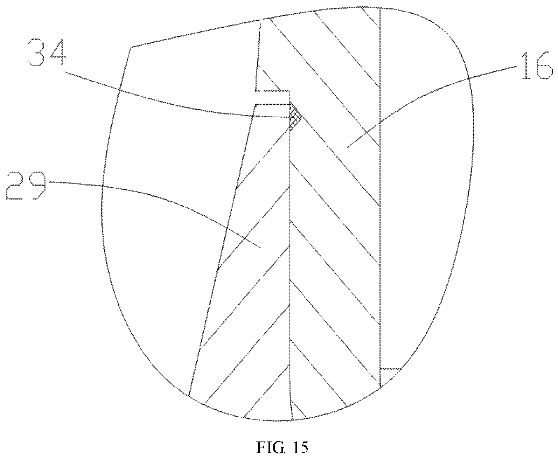

Further, referring to FIG. 14 and FIG. 15, the inner circumference surface of the built-in heating cavity 30 is provided with a plurality of elastic stoppers 34 that are spaced in the circumferential direction, so that the container body 16, when embedded in the built-in heating cavity 30, squeezes the plurality of elastic stoppers 34. In this way, the container body 16 squeezes the plurality of elastic stoppers 34 when it is embedded in the built-in heating cavity 30, thereby increasing the friction to prevent the container body from shaking or sliding.

Further, the plurality of elastic stoppers 34 may be evenly spaced in the circumferential direction. In addition, the plurality of elastic stoppers 34 may be separate components connected to the container body 16, or may be integrally molded with the container body 16. In some embodiments, the material of the plurality of elastic stoppers 34 may be a material with elastic properties, for example silica gel, rubber such as soft rubber or non-porous foam. In some embodiments, the elastic stoppers 34 may be evenly distributed at the entrance of the internal cavity of the container, and the number of the elastic stoppers may be 1, 2, 3 or more. In some embodiments, the structure of the plurality of elastic stoppers 34 may be a claw, hemisphere or block.

Further, referring to FIG. 14, in an embodiment, a horizontal inner bottom surface 35 and a vertical ring-shaped inner side surface 36 of the built-in heating cavity 30 are connected by a ring-shaped concave cambered inner surface 31 that is concave inward, and the concave cambered inner surface 31 is in contact with the convex cambered outer surface 28 of the convex cambered wall 27 of the container body 16 with the shapes thereof being matched. Accordingly, the convex cambered outer surface 28 and the concave cambered inner surface 31 that are contacted and matched in shapes can make the heating of the container body 16 more uniform and improve the heating efficiency.

Further, the container device may be a container device of any type. For example, in an embodiment, the container device may be a humidifier.

Further, referring to FIG. 6, a power device 37 is provided in the heating base 29. The power device 37 communicates with the container internal cavity 21 through a gas passage 38 to inject air into the container internal cavity 21 so that the container device functions as a ventilator. For example, the power device 37 communicates with the air intake passage 20 through the gas passage 38, and further communicates with the container internal cavity 21. In this way, the pressure in the container internal cavity may be increased when the power device 37 runs, so that the steam in the container internal cavity can be quickly expelled to enable the container device to function as a ventilator.

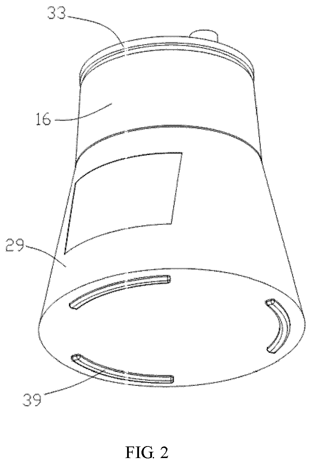

Further, referring to FIG. 2, the bottom surface of the heating base 29 is provided with a plurality of anti-skid supporting pads 39 evenly spaced in the circumferential direction. Thus, when the container device is placed on a tilted plane, the container device can be prevented from slipping and the stability of the container device can be improved.

Finally, the present disclosure provides a ventilation therapy apparatus including any container device described above. Accordingly, the overall performance of the ventilation therapy apparatus is improved. The ventilation therapy apparatus may be a ventilator or a high-flow oxygen therapy equipment.

In addition, it should be noted that the specific technical features described in the above-mentioned specific embodiment can be combined in any suitable manner as long as there is no contradiction. In order to avoid unnecessary repetition, various possible combinations will not be described further in the present disclosure.

## Claims

1. A cover opening and closing member, comprising: a connection rod (1), a cover universal connector (2), a supporting universal joint (3) and a pendulum (4), wherein,
the supporting universal joint (3) is configured to be in universal rotation fit with a supporting universal cavity (9) on a supporting frame (8) in a container internal cavity of a container body; wherein the cover universal connector (2), the supporting universal joint (3) and the pendulum (4) are sequentially arranged on the connection rod (1) at intervals,
**characterized in that** the cover universal connector (2) is configured to be in universal rotation fit with a universal connection portion (5) on a container cover, the cover universal connector (2) comprises a first universal rotation mating surface (6) that is in universal rotation fit with a second universal rotation mating surface (7) of the universal connection portion (5).

2. The cover opening and closing member according to claim 1, comprising at least one of the following forms:
in form 1, each of the cover universal connector (2), the supporting universal joint (3) and the pendulum (4) is a sphere, and the first universal rotation mating surface (6) is a spherical surface;
in form 2, the cover universal connector (2) and the pendulum (4) are respectively disposed at both ends of the connection rod (1);
in form 3, the pendulum (4) is provided with a heater receiving cavity for receiving a heater that is configured to enable the pendulum to heat water in the container internal cavity.

3. The cover opening and closing member according to claim 2, wherein in form 3, a wire routing passage (10) extending in an axial direction is formed inside the connection rod (1), and a wire passing passage (11) is formed in the cover universal connector (2), and the heater receiving cavity, the wire passing passage (11) and the wire routing passage (10) communicate with each other.

4. The cover opening and closing member according to claim 2 or 3, wherein in form 3, a heater is provided in the heater receiving cavity.

5. A combination of the cover opening and closing member according to any one of claims 1 to 4 and a container cover, comprising a cover body (12) configured with a universal connection portion (5) that is enabled to be in universal rotation fit with the cover universal connector (2) of the cover opening and closing member, wherein the universal connection portion (5) comprises the second universal rotation mating surface (7) that is in universal rotation fit with the first universal rotation mating surface (6) of the cover universal connector (2) of the cover opening and closing member.

6. The combination according to claim 5, wherein the container cover comprises at least one of:
situation 1, the universal connection portion (5) is a spherical cavity (14) with an inlet/outlet (13), and the second universal rotation mating surface (7) is an inner spherical surface of the spherical cavity (14);
situation 2, the universal connection portion (5) comprises a wire passing passage;
situation 3, circumferential edges of the cover body (12) comprise a ring-shaped convex spherical segment (15), and the ring-shaped convex spherical segment (15) is configured to be in universal sliding fit with a ring-shaped concave spherical segment (17) on edges of a cover port of a container body (16);
situation 4, the cover body (12) comprises an air exhaust passage (19) and an air intake passage (20) for feeding air into the container internal cavity of the container body.

7. The combination according to claim 6, wherein in situation 3, the circumferential edges of the cover body (12) comprise an annular sealing ring (18), and the annular sealing ring (18) comprises the ring-shaped convex spherical segment (15).

8. A combination of the cover opening and closing member according to any one of claims 1 to 4 and a container body, comprising a container internal cavity (21) with a cover port, wherein a supporting frame (8) is arranged in the container internal cavity (21), the supporting frame (8) comprises a supporting universal cavity (9) that is enabled to be in universal rotation fit with the supporting universal joint (3) of the cover opening and closing member, the supporting universal cavity (9) comprises openings at both ends in an axial direction.

9. The combination according to claim 8, wherein the container body comprises at least one of the following structures:
structure one, the supporting universal cavity (9) is a spherical frustum cavity;
structure two, the supporting frame (8) comprises a plurality of radial supporting rods (22) that are spaced from each other in a circumferential direction, wherein radial inner ends of the plurality of radial supporting rods (22) are connected together to form a center connection portion (23), and radial outer ends of the plurality of radial supporting rods (22) are connected to an inner surface of the container internal cavity (21), wherein the supporting universal cavity (9) is formed on the center connection portion (23);
structure three, edges of the openings at two ends of the supporting universal cavity (9) are respectively formed with a cutout recess (24);
structure four, edges of the cover port comprise a ring-shaped concave spherical segment (17) that is configured to be in universal sliding fit with the ring-shaped convex spherical segment (15) at the circumferential edges of the container cover;
structure five, a bottom wall (25) and a ring-shaped side wall (26) of the container body (16) are connected by a ring-shaped convex cambered wall (27), and the convex cambered wall (27) comprises a convex cambered outer surface (28) that is configured to be in contact with a concave cambered inner surface (31) of a built-in heating cavity (30) on a heating base (29) of the container, and the convex cambered outer surface (28) and the concave cambered inner surface (31) are matched in shapes.

10. A container device, comprising the cover opening and closing member (32) according to any one of claims 1 to 4, a container cover (33), and a container body (16), wherein,
the first universal rotation mating surface (6) is in universal rotation fit with the second universal rotation mating surface (7);
the supporting universal joint (3) is in universal rotation fit with the supporting universal cavity (9);
wherein the cover opening and closing member is enabled to be at a vertical position to cover the container cover (33) on the cover port of the container body (16);
when the container body (16) is tilted in any direction, the connection rod (1) is always kept at the vertical position by the pendulum (4), the cover universal connector (2) is enabled to universally rotate relative to the universal connection portion (5) through universal support of the supporting frame (8), so as to drive the container cover (33) to slide upward relative to the cover port of the container body (16) to be opened.

11. The container device according to claim 10, wherein the container device comprises a heating base (29), and the heating base (29) comprises a built-in heating cavity (30) with an upward opening, the container body (16) is enabled to be embedded downward in the built-in heating cavity (30).

12. The container device according to claim 11, wherein an inner circumference surface of the built-in heating cavity (30) is provided with a plurality of elastic stoppers (34) that are spaced in the circumferential direction, the container body (16) is enabled to, when embedded in the built-in heating cavity (30), squeeze the plurality of elastic stoppers (34).

13. The container device according to claim 11 or 12, wherein a horizontal inner bottom surface (35) and a vertical ring-shaped inner side surface (36) of the built-in heating cavity (30) are connected by a ring-shaped concave cambered inner surface (31) that is concave inward, and the concave cambered inner surface (31) is in contact with the convex cambered outer surface (28) of the convex cambered wall (27) of the container body (16), and the concave cambered inner surface (31) is matched with the convex cambered outer surface (28) in shapes.

14. The container device according to any one of claims 10 to 13, wherein the container device is a humidifier; or,
a power device (37) is provided in the heating base (29) of the container device, the power device (37) communicates with the container internal cavity (21) through a gas passage (38) to supply air into the container internal cavity (21) so that the container device functions as a ventilator.

15. A ventilation therapy apparatus comprising the container device according to any one of claims 10 to 14.

## Patentansprüche

1. Element zum Öffnen und Schließen einer Abdeckung, das umfasst: eine Verbindungsstange (1), einen Abdeckungs-Universalverbinder (2), ein tragendes Universalgelenk (3) sowie ein Pendel (4), wobei
das tragende Universalgelenk (3) so ausgeführt ist, dass es in Universal-Rotationspassung mit einem tragenden Universal-Hohlraum (9) an einem tragenden Rahmen (8) in einem Behälter-Innenhohlraum eines Behälterkörpers ist; wobei der Abdeckungs-Universalverbinder (2), das tragende Universalgelenk (3) und das Pendel (4) in Abständen aufeinanderfolgend an der Verbindungsstange (1) angeordnet sind,
**dadurch gekennzeichnet, dass** der Abdeckungs-Universalverbinder (2) so ausgeführt ist, dass er in Universal-Rotationspassung mit einem Universal-Verbindungsabschnitt (5) an einer Behälterabdeckung ist, wobei der Abdeckungs-Universalverbinder (2) eine erste Universal-Rotationspassfläche (6) umfasst, die in Universal-Rotationspassung mit einer zweiten Universal-Rotationspassfläche (7) des Universal-Verbindungsabschnitts (5) ist.

2. Element zum Öffnen und Schließen einer Abdeckung nach Anspruch 1, das wenigstens eine der folgenden Formen umfasst:
Form 1, bei der der Abdeckungs-Universalverbinder (2), das tragende Universalgelenk (3) und das Pendel (4) jeweils eine Kugel sind und die erste Universal-Rotationspassfläche (6) eine sphärische Fläche ist;
Form 2, bei der der Abdeckungs-Universalverbinder (2) und das Pendel (4) jeweils an beiden Enden der Verbindungsstange (1) angeordnet sind;
Form 3, bei der das Pendel (4) mit einem Hohlraum zum Aufnehmen einer Heizeinrichtung versehen ist, der eine Heizeinrichtung aufnimmt, die das Pendel befähigt, Wasser in dem Behälter-Innenraum zu erhitzen.

3. Element zum Öffnen und Schließen einer Abdeckung nach Anspruch 2, wobei in Form 3 ein Draht-Führungskanal (10), der in einer axialen Richtung verläuft, im Inneren der Verbindungsstange (1) ausgebildet ist, und ein Draht-Durchlasskanal (11) in dem Abdeckungs-Universalverbinder (2) ausgebildet ist, und der Hohlraum zum Aufnehmen einer Heizeinrichtung, der Draht-Durchlasskanal (11) und der Draht-Führungskanal (10) miteinander in Verbindung stehen.

4. Element zum Öffnen und Schließen einer Abdeckung nach Anspruch 2 oder 3, wobei in Form 3 eine Heizeinrichtung in dem Raum zum Aufnehmen einer Heizeinrichtung vorhanden ist.

5. Kombination aus dem Element zum Öffnen und Schließen einer Abdeckung nach einem der Ansprüche 1 bis 4 und einer Behälter-Abdeckung, umfassend einen Abdeckungskörper (12), der mit einem Universal-Verbindungsabschnitt (5) ausgeführt ist, der in Universal-Rotationspassung mit dem Abdeckungs-Universalverbinder (2) des Elementes zum Öffnen und Schließen einer Abdeckung sein kann, wobei der Universal-Verbindungsabschnitt(5) die zweite Universal-Rotationspassfläche (7) umfasst, die in Universal-Rotationspassung mit der ersten Rotationspassfläche (6) des Abdeckungs-Universalverbinders (2) des Elementes zum Öffnen und Schließen einer Abdeckung ist.

6. Kombination nach Anspruch 5, wobei die Behälterabdeckung wenigstens eine der folgenden Situationen umfasst:
Situation 1, in der der Universal-Verbindungsabschnitt (5) ein sphärischer Hohlraum (14) mit einem Einlass/Auslass (13) ist und die zweite Universal-Rotationspassfläche (7) eine sphärische Innenfläche des sphärischen Hohlraums (14) ist;
Situation 2, in der der Universal-Verbindungsabschnitt (5) einen Draht-Durchlasskanal umfasst;
Situation 3, in der Umfangsränder des Abdeckungskörpers (12) ein ringförmiges konvexe sphärisches Segment (15) umfassen und das ringförmige konvexe sphärische Segment (15) so ausgeführt ist, dass es in Universal-Gleitpassung mit einem ringförmigen konkaven sphärischen Segment (17) an Rändern einer Abdeckungsöffnung eines Behälterkörpers (16) ist;
Situation 4, in der der Abdeckungskörper (12) einen Luftauslasskanal (19) sowie einen Lufteinlasskanal (20) zum Einleiten von Luft in den Behälter-Innenhohlraum des Behälterkörpers umfasst.

7. Kombination nach Anspruch 6, wobei in Situation 3 die Umfangsränder des Abdeckungskörpers (12) einen Dichtungsring (18) umfassen und der Dichtungsring (18) das ringförmige konvexe sphärische Segment (15) umfasst.

8. Kombination aus dem Element zum Öffnen und Schließen einer Abdeckung nach einem der Ansprüche 1 bis 4 und einem Behälterkörper, umfassend einen Behälter-Innenhohlraum (21) mit einer Abdeckungsöffnung, wobei ein tragender Rahmen (8) in dem Behälter-Innenhohlraum (21) angeordnet ist, der tragende Rahmen (8) einen tragenden Universal-Hohlraum (9), der in Universal-Rotationspassung mit dem tragenden Universalgelenk (3) des Elementes zum Öffnen und Schließen einer Abdeckung sein kann, wobei der tragende Universal-Hohlraum (9) Öffnungen an beiden Enden in einer axialen Richtung umfasst.

9. Kombination nach Anspruch 8, wobei der Behälterkörper wenigstens eine der folgenden Strukturen umfasst:
Struktur 1, bei der der tragende Universal-Hohlraum (9) ein Kugelsegment-Hohlraum ist;
Struktur 2, bei der der tragende Rahmen (8) eine Vielzahl radialer Stützstangen (22) umfasst, die in einer Umfangsrichtung voneinander beabstandet sind, wobei radiale innere Enden der Vielzahl radialer Stützstangen (22) miteinander verbunden sind und einen mittigen Verbindungsabschnitt (23) bilden, und radiale äußere Enden der Vielzahl radialer Stützstangen (22) mit einer Innenfläche des Behälter-Innenhohlraums (21) verbunden sind, wobei der tragende Universal-Hohlraum (9) an dem mittigen Verbindungsabschnitt (23) ausgebildet ist;
Struktur 3, bei der Ränder der Öffnungen an zwei Enden des tragenden Universal-Hohlraums (9) jeweils mit einer ausgeschnittenen Aussparung (24) versehen sind;
Struktur 4, bei der Ränder der Abdeckungsöffnung ein ringförmiges konkaves sphärisches Segment (17) umfassen, das so ausgeführt ist, dass es in Universal-Gleitpassung mit dem ringförmigen konvexen sphärischen Segment (15) an den Umfangsrändern der Behälterabdeckung ist;
Struktur 5, bei der eine Bodenwand (25) und eine ringförmige Seitenwand (26) des Behälterkörpers (16) über eine ringförmige konvexe gewölbte Wand (27) verbunden sind, und die konvexe gewölbte Wand (27) eine konvexe gewölbte Außenfläche (28) umfasst, die so ausgeführt ist, dass sie in Kontakt mit einer konkaven gewölbten Innenfläche (31) eines eingebauten Heiz-Hohlraums (30) an einem Heizsockel (29) des Behälters sind, und die Formen der konvexen gewölbten Außenfläche (28) und der konkaven gewölbten Innenfläche (31) zueinander passen.

10. Behältervorrichtung, die das Element (32) zum Öffnen und Schließen einer Abdeckung nach einem der Ansprüche 1 bis 4, eine Behälterabdeckung (33) sowie einen Behälterkörper (16) umfasst, wobei
die erste Universal-Rotationspassfläche (6) in Universal-Rotationspassung mit der zweiten Universal-Rotationspassfläche (7) ist;
das tragende Universalgelenk (3) in Universal-Rotationspassung mit dem tragenden Universal-Hohlraum (9) ist;
wobei das Element zum Öffnen und Schließen einer Abdeckung sich an einer vertikalen Position zum Abdecken der Behälterabdeckung (33) an der Abdeckungsöffnung des Behälterkörpers (16) befinden kann;
wenn der Behälterkörper (16) in einer beliebigen Richtung geneigt wird, die Verbindungsstange (1) durch das Pendel (4) stets an der vertikalen Position gehalten wird, der Abdeckungs-Universal Verbinder (2) über Universallagerung des tragenden Rahmens (8) universal relativ zu dem Universal-Verbindungsabschnitt (5) rotieren kann, so dass die Behälterabdeckung (33) so bewegt wird, dass sie relativ zu der Abdeckungsöffnung des Behälterkörpers (16) nach oben geschoben und geöffnet wird.

11. Behältervorrichtung nach Anspruch 10, wobei die Behältervorrichtung einen Heizsockel (29) umfasst und der Heizsockel (29) einen eingebauten Heiz-Hohlraum (30) mit einer nach oben gerichteten Öffnung umfasst, und der Behälterkörper (16) nach unten in den eingebauten Heiz-Hohlraum (30) eingebettet werden kann.

12. Behältervorrichtung nach Anspruch 11, wobei eine Innenumfangsfläche des eingebauten Heiz-Hohlraums (30) mit einer Vielzahl elastischer Stopper (34) versehen ist, die in der Umfangsrichtung beabstandet sind, und der Behälterkörper (16), wenn er in den eingebauten Heiz-Hohlraum (30) eingebettet ist, die Vielzahl elastischer Stopper (34) zusammendrücken kann.

13. Behältervorrichtung nach Anspruch 11 oder 12, wobei eine horizontale Boden-Innenfläche (35) und eine vertikale ringförmige Seiten-Innenfläche (36) des eingebauten Heiz-Hohlraums (30) über eine ringförmige konkave gewölbte Innenfläche (31) verbunden sind, die nach innen konkav ist, und die konkave gewölbte Innenfläche (31) in Kontakt mit der konvexen gewölbten Außenfläche (28) der konvexen gewölbten Wand (27) des Behälterkörpers (16) ist, und die Formen der konkaven gewölbten Innenfläche (31) und der konvexen gewölbten Außenfläche (28) zueinander passen.

14. Behältervorrichtung nach einem der Ansprüche 10 bis 13, wobei die Behältervorrichtung ein Befeuchter ist; oder
eine Antriebsvorrichtung (37) in dem Heizsockel (29) der Behältervorrichtung vorhanden ist, wobei die Antriebsvorrichtung (37) mit dem Behälter-Innenhohlraum (21) über einen Gaskanal (38) zum Einleiten von Luft in den Behälter-Innenhohlraum (21) in Verbindung steht, so dass die Behältervorrichtung als ein Ventilator wirkt.

15. Beatmungstherapiegerät, das die Behältervorrichtung nach einem der Ansprüche 10 bis 14 umfasst.

## Revendications

1. Élément d'ouverture et de fermeture de couvercle, comprenant : une tige de connexion (1), un connecteur universel de couvercle (2), un joint universel de support (3) et un pendule (4), dans lequel,
le joint universel de support (3) est configuré pour être en ajustement par rotation universelle avec une cavité universelle de support (9) sur un cadre de support (8) dans une cavité interne de récipient d'un corps de récipient ; dans lequel le connecteur universel de couvercle (2), le joint universel de support (3) et le pendule (4) sont disposés séquentiellement sur la tige de connexion (1) selon des intervalles,
**caractérisé en ce que** le connecteur universel de couvercle (2) est configuré pour être en ajustement par rotation universelle avec une partie de connexion universelle (5) sur un couvercle de récipient, le connecteur universel de couvercle (2) comprend une première surface d'accouplement par rotation universelle (6) qui est en ajustement par rotation universelle avec une seconde surface d'accouplement par rotation universelle (7) de la partie de connexion universelle (5).

2. Élément d'ouverture et de fermeture de couvercle selon la revendication 1, comprenant au moins une des formes suivantes :
dans la forme 1, chacun du connecteur universel de couvercle (2), du joint universel de support (3) et du pendule (4) est une sphère, et la première surface d'accouplement par rotation universelle (6) est une surface sphérique ;
dans la forme 2, le connecteur universel de couvercle (2) et le pendule (4) sont respectivement disposés aux deux extrémités de la tige de connexion (1) ;
dans la forme 3, le pendule (4) est équipé d'une cavité réceptrice de chauffage afin de recevoir un chauffage qui est configuré pour permettre au pendule de chauffer de l'eau dans la cavité interne de récipient.

3. Élément d'ouverture et de fermeture de couvercle selon la revendication 2, dans lequel, dans la forme 3, un passage d'acheminement de fil (10) s'étendant dans une direction axiale est formé à l'intérieur de la tige de connexion (1), et un passage de transmission de fil (11) est formé dans le connecteur universel de couvercle (2), et la cavité réceptrice de chauffage, le passage de transmission de fil (11) et le passage d'acheminement de fil (10) communiquent les uns avec les autres.

4. Élément d'ouverture et de fermeture de couvercle selon la revendication 2 ou 3, dans lequel, dans la forme 3, un chauffage est agencé dans la cavité réceptrice de chauffage.

5. Combinaison constituée de l'élément d'ouverture et de fermeture de couvercle selon l'une quelconque des revendications 1 à 4 et d'un couvercle de récipient, comprenant un corps de couvercle (12) configuré avec une partie de connexion universelle (5) qui est adaptée pour être en ajustement par rotation universelle avec le connecteur universel de couvercle (2) de l'élément d'ouverture et de fermeture de couvercle, dans laquelle la partie de connexion universelle (5) comprend la seconde surface d'accouplement par rotation universelle (7) qui est en ajustement par rotation universelle avec la première surface d'accouplement par rotation universelle (6) du connecteur universel de couvercle (2) de l'élément d'ouverture et de fermeture de couvercle.

6. Combinaison selon la revendication 5, dans laquelle le couvercle de récipient comprend au moins l'une parmi :
situation 1, la partie de connexion universelle (5) est une cavité sphérique (14) avec une entrée/sortie (13), et la seconde surface d'accouplement par rotation universelle (7) est une surface sphérique intérieure de la cavité sphérique (14) ;
situation 2, la partie de connexion universelle (5) comprend un passage de transmission de fil ;
situation 3, des bords circonférentiels du corps de couvercle (12) comprennent un segment sphérique convexe en forme d'anneau (15), et le segment sphérique convexe en forme d'anneau (15) est configuré pour être en ajustement coulissant universel avec un segment sphérique concave en forme d'anneau (17) sur des bords d'un orifice de couvercle d'un corps de récipient (16) ;
situation 4, le corps de couvercle (12) comprend un passage d'échappement d'air (19) et un passage d'admission d'air (20) pour l'alimentation en air de la cavité interne de récipient du corps de récipient.

7. Combinaison selon la revendication 6, dans laquelle, dans la situation 3, les bords circonférentiels du corps de couvercle (12) comprennent un anneau d'étanchéité annulaire (18), et l'anneau d'étanchéité annulaire (18) comprend le segment sphérique convexe en forme d'anneau (15).

8. Combinaison constituée de l'élément d'ouverture et de fermeture de couvercle selon l'une quelconque des revendications 1 à 4 et d'un corps de récipient, comprenant une cavité interne de récipient (21) avec un orifice de couvercle, dans laquelle un cadre de support (8) est agencé dans la cavité interne de récipient (21), le cadre de support (8) comprend une cavité universelle de support (9) qui est adaptée pour être en ajustement par rotation universelle avec le joint universel de support (3) de l'élément d'ouverture et de fermeture de couvercle, la cavité universelle de support (9) comprend des ouvertures aux deux extrémités dans une direction axiale.

9. Combinaison selon la revendication 8, dans laquelle le corps de récipient comprend au moins l'une des structures suivantes :
structure un, la cavité universelle de support (9), est une cavité sphérique tronconique ;
structure deux, le cadre de support (8) comprend une pluralité de tiges de support radiales (22) qui sont espacées les unes des autres dans une direction circonférentielle, dans laquelle des extrémités intérieures radiales de la pluralité de tiges de support radiales (22) sont connectées ensemble pour former une partie de connexion centrale (23), et des extrémités extérieures radiales de la pluralité de tiges de support radiales (22) sont connectées à une surface intérieure de la cavité interne de récipient (21), dans laquelle la cavité universelle de support (9) est formée sur la partie de connexion centrale (23) ;
structure trois, des bords des ouvertures à deux extrémités de la cavité universelle de support (9) sont respectivement munis d'un évidement découpé (24) ;
structure quatre, des bords de l'orifice de couvercle comprennent un segment sphérique concave en forme d'anneau (17) qui est configuré pour être en ajustement coulissant universel avec le segment sphérique convexe en forme d'anneau (15) au niveau des bords circonférentiels du couvercle de récipient ;
structure cinq, une paroi inférieure (25) et une paroi latérale en forme d'anneau (26) du corps de récipient (16) sont connectées par une paroi cambrée convexe en forme d'anneau (27), et la paroi cambrée convexe (27) comprend une surface extérieure cambrée convexe (28) qui est configurée pour être en contact avec une surface intérieure cambrée concave (31) d'une cavité de chauffage intégrée (30) sur une base chauffante (29) du récipient, et la surface extérieure cambrée convexe (28) et la surface intérieure cambrée concave (31) coïncident en termes de formes.

10. Dispositif de récipient, comprenant l'élément d'ouverture et de fermeture de couvercle (32) selon l'une quelconque des revendications 1 à 4, un couvercle de récipient (33) et un corps de récipient (16), dans lequel
la première surface d'accouplement par rotation universelle (6) est en ajustement par rotation universelle avec la seconde surface d'accouplement par rotation universelle (7) ;
le joint universel de support (3) est en ajustement par rotation universelle avec la cavité universelle de support (9) ;
dans lequel l'élément d'ouverture et de fermeture de couvercle est adapté pour être à une position verticale pour recouvrir le couvercle de récipient (33) sur l'orifice de couvercle du corps de récipient (16) ;
lorsque le corps de récipient (16) est incliné dans une quelconque direction, la tige de connexion (1) est toujours maintenue à la position verticale par le pendule (4), le connecteur universel de couvercle (2) est adapté pour tourner universellement par rapport à la partie de connexion universelle (5) via le support universel du cadre de support (8), de manière à entrainer le couvercle du récipient (33) pour qu'il coulisse vers le haut par rapport à l'orifice de couvercle du corps de récipient (16) à ouvrir.

11. Dispositif de récipient selon la revendication 10, dans lequel le dispositif de récipient comprend une base chauffante (29) et la base chauffante (29) comprend une cavité chauffante intégrée (30) avec une ouverture vers le haut, le corps de récipient (16) est adapté pour être encastré vers le bas dans la cavité chauffante intégrée (30).

12. Dispositif de récipient selon la revendication 11, dans lequel une surface de circonférence intérieure de la cavité chauffante intégrée (30) est équipée d'une pluralité de butées élastiques (34) qui sont espacés dans la direction circonférentielle, le corps de récipient (16) est adapté, lorsqu'il est encastré dans la cavité chauffante intégrée (30), pour comprimer la pluralité de butées élastiques (34).

13. Dispositif de récipient selon la revendication 11 ou 12, dans lequel une surface inférieure intérieure horizontale (35) et une surface latérale intérieure verticale en forme d'anneau (36) de la cavité chauffante intégrée (30) sont connectées par une surface intérieure cambrée concave en forme d'anneau (31) qui est concave vers l'intérieur, et la surface intérieure cambrée concave (31) est en contact avec la surface extérieure cambrée convexe (28) de la paroi cambrée convexe (27) du corps de récipient (16), et la surface intérieure cambrée concave (31) coïncide avec la surface extérieure cambrée convexe (28) en termes de formes.

14. Dispositif de récipient selon l'une quelconque des revendications 10 à 13, dans lequel le dispositif de récipient est un humidificateur ; ou,
un dispositif d'alimentation en énergie (37) est agencé dans la base chauffante (29) du dispositif de récipient, le dispositif d'alimentation en énergie (37) communique avec la cavité interne de récipient (21) via un passage de gaz (38) pour fournir de l'air dans la cavité interne de récipient (21) de sorte que le dispositif de récipient fonctionne comme un ventilateur.

15. Appareil thérapeutique ventilatoire comprenant le dispositif de récipient selon l'une quelconque des revendications 10 à 14.
